# EUROPEAN PATENT SPECIFICATION

(11) **EP 3 053 628 B1**
(45) Date of publication and mention of the grant of the patent: **01.08.2018**
(21) Application number: 13895057.1
(22) Date of filing: 04.10.2013
(51) Int. Cl.: A61N 5/10, G21K 1/04

(54) **MULTILEAF COLLIMATOR, AND RADIATION THERAPY APPARATUS AND RADIATION THERAPY SYSTEM USING SAME**
MEHRBLATTKOLLIMATOR UND STRAHLENTHERAPIEVORRICHTUNG SOWIE STRAHLENTHERAPIESYSTEM DAMIT
COLLIMATEUR À LAMES MULTIPLES, ET APPAREIL DE RADIOTHÉRAPIE ET SYSTÈME DE RADIOTHÉRAPIE L'UTILISANT

(43) Date of publication of application: 10.08.2016
(73) Proprietor: Hitachi, Ltd., Tokyo 100-8280 (JP)
(72) Inventor: TAKAHASHI, Kunio, Tokyo 108-8215 (JP); MIYAMOTO, Akihiro, Tokyo 108-8215 (JP)
(74) Representative: Strehl Schübel-Hopf & Partner
(86) International application number: PCT/JP2013/077050
(87) International publication number: WO 2015/049787

(56) References cited:
- JP-A- H01 206 300
- JP-A- 2004 275 243
- JP-A- 2007 117 464
- JP-A- 2007 195 877
- US-A1- 2006 198 492
- US-A1- 2008 165 930

## Description

### Technical Field

The present invention relates to a multileaf collimator that controls the irradiation field of radiation radiated in order to perform radiation therapy, and a radiation therapy apparatus and a radiation therapy system using the same.

### Background Art

As one of therapies for tumors, there is radiation therapy in which an affected part is irradiated. In the radiation therapy, it is desired to irradiate an affected part efficiently while suppressing the dose of radiation (radiation dose) to a patient to be as low as possible.

Thus, multileaf collimators that control an irradiation field, that is, an irradiation area or irradiation shape of radiation, have been used.

In the multileaf collimators, sheet-like multiple leaves are provided side by side at intervals in their respective thickness directions within a frame. The respective leaves are provided so as to be movable in directions along their respective surfaces. Since a portion of the irradiation area of radiation is shielded by such respective leaves, the irradiation field of the radiation is controlled.

For example, PTL 1 discloses a configuration including a rack gear that is formed at an inner edge of an opening formed in each leaf, a pinion gear that meshes with the rack gear, and a motor that rotates the pinion gear rotate, as a driving mechanism that drives each leaf.

Here, driving shafts of the motors are inserted into the openings of the multiple leaves that are provided side by side, in a direction (the thickness directions of the respective leaves) in which the leaves are provided side by side. The rack gear provided at the tip of the driving shaft meshes with the rack gear formed at the inner edge of the opening of each Further, PTL 2 shows an MLC with leaves having slits into which shafts are inserted to support said leaves mechanically.Another shafts, contacting leaves on their sides, are used to drive the leaf movement.

### Citation List

### Patent Literature

[PTL 1] Japanese Patent No. (PTL 2) US 2006 198 492

### Summary of Invention

### Technical Problem

Meanwhile, the motors as described above are fixed to an outer peripheral surface of the frame that houses the multiple leaves. Then, the intervals between the outer peripheral surface (the attachment surface for the motors) of the frame and the respective leaves vary in every leaf. Therefore, the lengths of the shafts of the motors that drive the respective leaves vary in every leaf.

As a result, a number of types of shafts having different lengths should be prepared. For this reason, the types of parts that constitute the multileaf collimators are increased, which becomes a hindrance to suppression of part costs. Moreover, shafts having lengths according to the positions of the individual leaves should be prepared at the time of repair. That is, the number of parts to be stocked is increased, and this also becomes a hindrance to cost suppression.

An object of the invention is to provide a multileaf collimator in which the number of types of parts of the multileaf collimator can be reduced and it is possible to suppress manufacture costs and maintenance costs, and a radiation therapy apparatus and a radiation therapy system using the same.

### Solution to Problem

According to a first aspect related to the invention, a multileaf collimator includes a plurality of leaves that extend in a first direction; each leaf having a slit formed so as to pass therethrough in the thickness direction; frame that supports the plurality of leaves so that the plurality of leaves are individually advanceable and retractable in a state where the plurality of leaves are placed side by side in a second direction perpendicular to the first direction. The multileaf collimator further includes a plurality of shafts that extend in the second direction and are provided to correspond to the plurality of leaves, respectively; wherein one end of each shaft in inserted into the slit of the corresponding leaf; and a plurality of driving units that are respectively connected to first ends of the plurality shafts in directions of axial lines and rotate the shafts around the axial lines. The multileaf collimator further includes advance and retraction driving sections that are provided at second ends of the shafts in the directions of the axial lines, contact portions of the leaves, and advance and retract the leaves in the first direction by the rotation of the shafts around the axial lines. Additionally, in the multileaf collimator, at least some of the plurality of shafts have the same shaft length as each other, and the plurality of driving units are arranged so that the positions thereof in the second direction are shifted from each other according to the positions of the second ends of the plurality of shafts having the same length. Further, at least some of the plurality of driving units may be supported via spacers with respect to the frame, and the thicknesses of the spacers in the second direction may be set in a plurality of steps according to the positions of the driving units in the second direction.

The frame may include pedestal parts that fix the driving units, and the heights of the pedestal parts of the second direction may be set in a plurality of steps according to the positions of the driving units in the second direction.

The plurality of shafts may include shafts having at least two types of length.

The driving units may be arranged so that the projection dimensions thereof from the frame in the second direction become gradually larger from the outside of an attachment surface of the frame to which the plurality of driving units are attached toward a central side of the attachment surface.

The invention also encompasses a radiation therapy apparatus including the multileaf collimator; and a radiation irradiation device that radiates radiation.

The invention also encompasses a radiation therapy system including the radiation therapy apparatus ; and a control apparatus that controls the operation of the radiation therapy apparatus.

The invention is defined in the claims, other embodiments being merely exemplary.

### Advantageous Effects of Invention

According to the above-described multileaf collimator, radiation therapy apparatus, and radiation therapy system, the number of types of parts of the multileaf collimator can be reduced, and it is possible to suppress manufacture costs and maintenance costs.

### Brief Description of Drawings

Fig. 1 is a view illustrating a functional configuration of a radiation therapy system in a first embodiment of the invention.
Fig. 2 is a perspective view illustrating a schematic configuration of a radiation therapy apparatus that constitutes the radiation therapy system in the first embodiment of the invention.
Fig. 3 is a sectional view illustrating a radiation irradiation device that constitutes the radiation therapy apparatus in the first embodiment of the invention.
Fig. 4 is a perspective view illustrating the external appearance of a multileaf collimator that constitutes a portion of the radiation irradiation device in the first embodiment of the invention.
Fig. 5 is a width-direction sectional view of the multileaf collimator in the first embodiment of the invention.
Fig. 6 is a sectional view orthogonal to a thickness direction of each leaf of the multileaf collimator in the first embodiment of the invention.
Fig. 7 is a perspective view illustrating leaves and a drive device that drives the leaves, in the first embodiment of the invention.
Fig. 8 is a perspective view illustrating the configuration of a drive device in the first embodiment of the invention.
Fig. 9 is a sectional view illustrating the arrangement structure of drive devices with respect to the leaves in the first embodiment of the invention.
Fig. 10 is a sectional view illustrating the arrangement structure of the drive devices with respect to the leaves in a modification example of the first embodiment of the invention.
Fig. 11 is a sectional view illustrating the arrangement structure of the drive devices with respect to the leaves of a multileaf collimator in a second embodiment.
Fig. 12 is a sectional view illustrating the arrangement structure of the drive devices with respect to the leaves of a multileaf collimator in a third embodiment.
Fig. 13 is a perspective view of main parts of the multileaf collimator in the third embodiment.

### Description of Embodiments

### (First Embodiment)

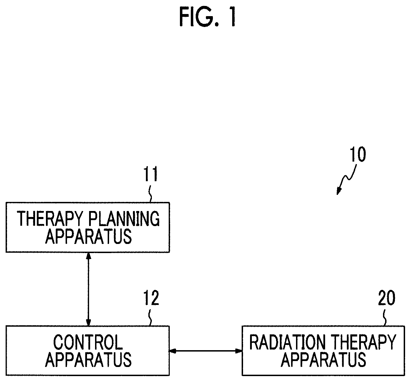

Fig. 1 is a view illustrating a functional configuration of a radiation therapy system 10 in a first embodiment of the invention.

As illustrated in Fig. 1, the radiation therapy system 10 includes a therapy planning apparatus 11, a control apparatus 12, and a radiation therapy apparatus 20.

In the therapy planning apparatus 11, properties (the strength, time, angle, position, radiation area, and the like of radiation to be radiated to a patient) of radiation to be radiated to preset and a patient, which are preset according to the contents of radiation therapy to be performed to a patient, are input from the outside. In the therapy planning apparatus 11, various control parameter values for radiating radiation corresponding to the input properties of the radiation are output to the control apparatus 12.

The control apparatus 12 controls the operation of the radiation therapy apparatus 20 on the basis of the various parameter values produced by the therapy planning apparatus 11. The control apparatus 12 is a computer apparatus, such as a personal computer, which executes processing on the basis of a predetermined program. The control apparatus 12 is connected to the radiation therapy apparatus 20 via a wireless or wired communication line so that information can be transmitted bidirectionally.

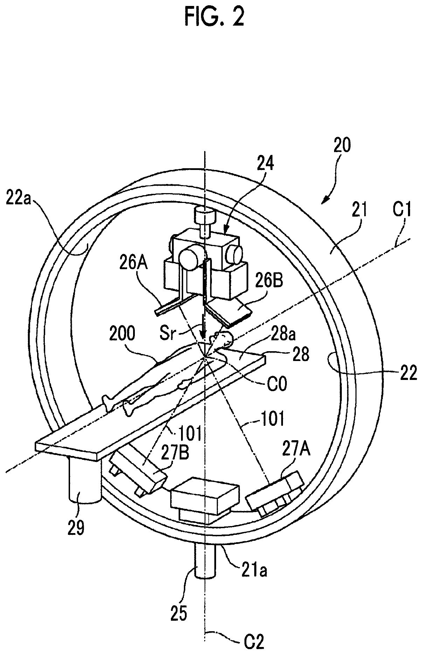

Fig. 2 is a perspective view illustrating a schematic configuration of the radiation therapy apparatus 20 that constitutes the radiation therapy system 10.

As illustrated in Fig. 2, the radiation therapy apparatus 20 includes a ring frame 21, a traveling gantry 22, and a radiation irradiation device 24.

The ring frame 21 is formed in a tubular shape having a circular section. The ring frame 21 is disposed so that a central axis C1 substantially faces a horizontal direction. The ring frame 21 has a downward extending rotating shaft 25 formed integrally with an outer peripheral surface of a lower end 21a. The rotating shaft 25 is supported by a base (not illustrated) so as to be rotatable around a central axis C2 of the rotating shaft 25. The rotating shaft 25 is rotated around the central axis C2 of the rotating shaft 25 by a swiveling driving mechanism (not illustrated). Accordingly, the ring frame 21 is made rotatable around the central axis C2 of the rotating shaft 25, that is, around a vertical axis.

The traveling gantry 22 is formed in a tubular shape having a circular section. The traveling gantry 22 is arranged on an inner peripheral side of the ring frame 21. The traveling gantry 22 is supported so as to be rotatable along an inner peripheral surface of the ring frame 21. Accordingly, the annular traveling gantry 22 is made rotatable around the central axis C1 extending in the horizontal direction. The traveling gantry 22 is made swivable around the central axis C1 by a gantry driving mechanism (not illustrated).

The radiation irradiation device 24 is controlled by the control apparatus 12, and radiates therapeutic radiation Sr. The radiation irradiation device 24 is supported by an inner peripheral surface 22a of the traveling gantry 22. The radiation irradiation device 24 is provided so that the therapeutic radiation Sr to be radiated passes through an isocenter C0 that is an intersection point between the central axis C2 of the rotational operation of the ring frame 21 and the central axis C1 of the rotational operation of the traveling gantry 22.

In this way, when the radiation irradiation device 24 is supported by the traveling gantry 22, the radiation irradiation device 24 radiates the therapeutic radiation Sr so as to pass through the isocenter C0 always irrespective of the rotational operation of the ring frame 21 around the central axis C2 and the rotational operation of the traveling gantry 22 around the central axis C1.

Additionally, the radiation therapy apparatus 20 includes diagnosing X-ray sources 26A and 26B, and sensor arrays 27A and 27B.

The diagnosing X-ray sources 26A and 26B are arranged on an inner peripheral side of the traveling gantry 22. The diagnosing X-ray sources 26A and 26B are arranged on both sides of the ring frame 21 in a circumferential direction with the center (the central axis C2 of the rotational operation of the ring frame 21) of the radiation therapy apparatus 20 interposed therebetween. The diagnosing X-ray sources 26A and 26B are controlled by the control apparatus 12 and radiate diagnosing X-rays 101 toward the isocenter C0. The diagnosing X-rays 101 are conic cone beams that widen in the shape of a cone from the point of origin of the diagnosing X-ray sources 26A and 26B.

The sensor arrays 27A and 27B are supported by the inner peripheral surface 22a of the traveling gantry 22. The sensor arrays 27A and 27B are provided so as to face the diagnosing X-ray sources 26A and 26B with the isocenter C0 interposed therebetween. The sensor arrays 27A and 27B receive the diagnosing X-rays 101 that are radiated from the diagnosing X-ray sources 26A and 26B and are transmitted through a photographic subject around the isocenter C0, and produce a transmission image of the photographic subject. The sensor arrays 27A and 27B include, for example, flat panel detectors (FPDs), X-ray image Intensifiers (II) or the like.

The radiation therapy apparatus 20 further includes a table 28 and a table drive device 29. A patient 200 to be treated by the radiation therapy system 10 lies on his/her side on an upper surface 28a of the table 28.

The table drive device 29 is controlled by the control apparatus 12 and moves the table 28. The table drive device 29 is supported by a base (not illustrated).

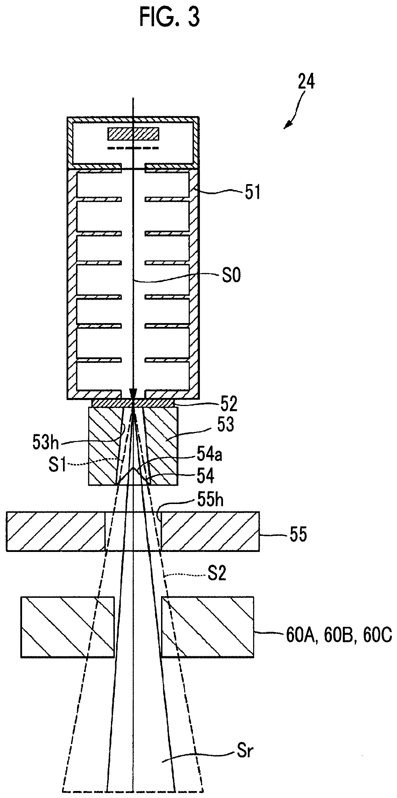

Fig. 3 is a sectional view illustrating the radiation irradiation device 24 that constitutes the radiation therapy apparatus 20.

As illustrated in Fig. 3, the radiation irradiation device 24 includes an electron beam accelerator 51, an X-ray target 52, a primary collimator 53, a flattening filter 54, a secondary collimator 55, and a multileaf collimator 60A.

The electron beam accelerator 51 irradiates the X-ray target 52 with an electron beam S0 produced by accelerates electrons.

The X-ray target 52 is formed of tungsten, a tungsten alloy, or the like. The X-ray target 52 emits radiation S1 if the target is irradiated with the electron beam S0.

The primary collimator 53 is formed of lead, tungsten, or the like. A through-hole 53h is formed in the primary collimator 53. The radiation S1 radiated from the X-ray target 52 passes through the through-hole 53h. The primary collimator 53 shields a portion of the radiation S1 so that the radiation S1 is not radiated by the through-hole 53h other than a desired portion.

The flattening filter 54 is formed of aluminum or the like. The flattening filter 54 is arranged on an outlet side of the through-hole 53h of the primary collimator 53. The flattening filter 54 has a substantially conical projection 54a on a side that faces the X-ray target 52. In the flattening filter 54, the shape of the projection 54a is designed so that a radiation dose in a plane perpendicular to a radial direction is substantially uniformly distributed after the radiation S1 has passed through the flattening filter 54.

The secondary collimator 55 is formed of lead, tungsten, or the like. The secondary collimator 55 includes a through-hole 55h at a central part therein. The secondary collimator 55 radiates only radiation S2 that has passed through the through-hole 55h, and shields a portion of the radiation S1.

By passing through the above-described primary collimator 53, the flattening filter 54, and the secondary collimator 55, a portion of the radiation S2 having uniform intensity distribution is shielded by the multileaf collimator 60A. The multileaf collimator 60A undergoes the control of the control apparatus 12, and produces the therapeutic radiation Sr that is set in the therapy planning apparatus 11 and is obtained according to the properties of radiation to be radiated to a patient.

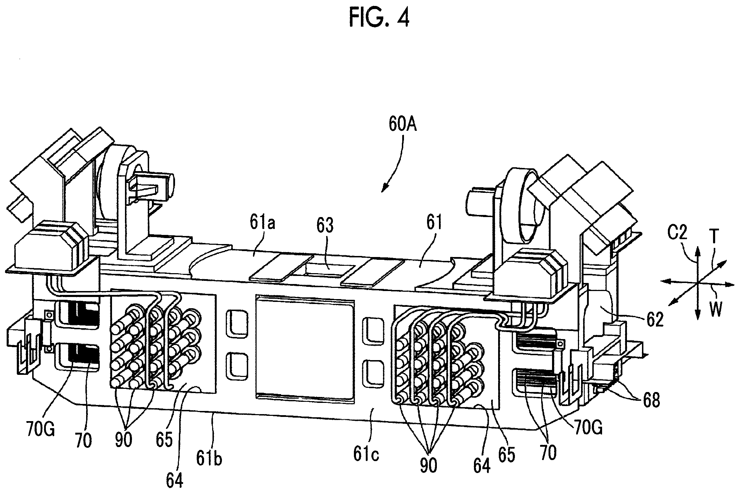

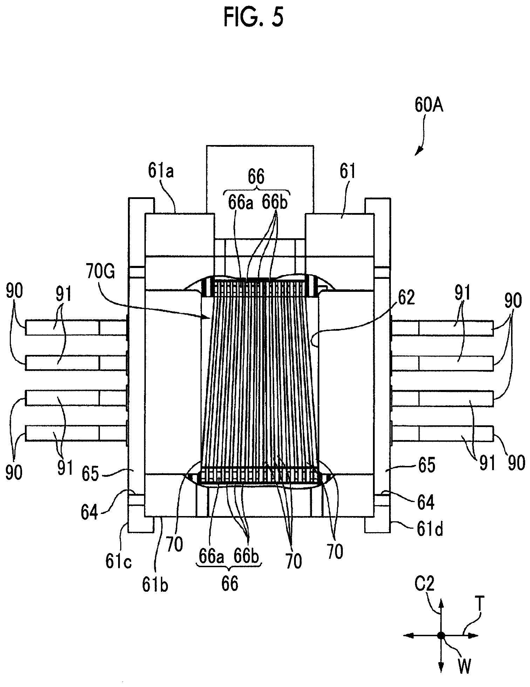

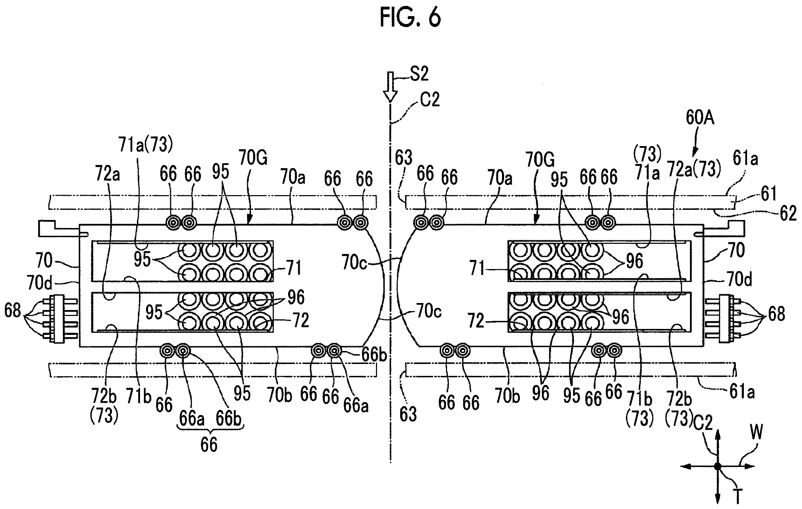

Fig. 4 is a perspective view illustrating the external appearance of the multileaf collimator 60A that constitutes a portion of the radiation irradiation device 24. Fig. 5 is a width-direction sectional view of the multileaf collimator 60A. Fig. 6 is a sectional view of the multileaf collimator 60A that is orthogonal to a second direction (hereinafter, referred to as a thickness direction T) that is a thickness direction of each leaf 70.

As illustrated in Figs. 4 to 6, the multileaf collimator 60A includes a frame 61, a plurality of leaves 70, and a drive device 90.

The frame 61 is formed in a substantially rectangular parallelepiped shape that is elongated in one direction. The frame 61 is arranged so that a first direction (hereinafter, referred to as a width direction W) that is a longitudinal direction of the frame is orthogonal to a radiation irradiation axis of the radiation irradiation device 24 extending along the central axis C2. A hollow leaf housing part 62 that is continuous in the width direction W is formed in the frame 61.

In the frame 61, openings 63, which pass through an outer peripheral side of the frame 61 and the leaf housing part 62, are formed in an upper surface 61a on a side that faces the radiation irradiation device 24 and a lower surface 61b opposite to the upper surface (in Fig. 4, only the opening 63 of the upper surface 61a is illustrated). The openings 63 are formed at central parts of the upper surface 61a and the lower surface 61b in the width direction W.

As illustrated in Figs. 4 and 5, in the frame 61, rectangular openings 64 and 64 are respectively formed in both side surfaces 61c and 61d orthogonal to the upper surface 61a and the lower surface 61b. The openings 64 and 64 are formed so as to be symmetrical to central parts of the side surface 61c or 61d in the width direction W. Rectangular base plates 65 are respectively mounted on the openings 64.

The leaves 70 are approximately formed in a substantially oblong shape. The leaves 70 are formed of, for example, tungsten, a tungsten alloy, or the like.

As illustrated in Fig. 5, the plurality of leaves 70 are placed side by side at intervals in the thickness direction T. A leaf group 70G is constituted of the plurality of leaves 70 that are placed side by side. In this embodiment, the leaf group 70G is constituted by, for example, thirty leaves 70. As illustrated in Fig. 4, two such leaf groups 70G are arranged in a pair within the leaf housing part 62 within the frame 61 so as to face each other with the central part of the frame 61 in the width direction W interposed therebetween.

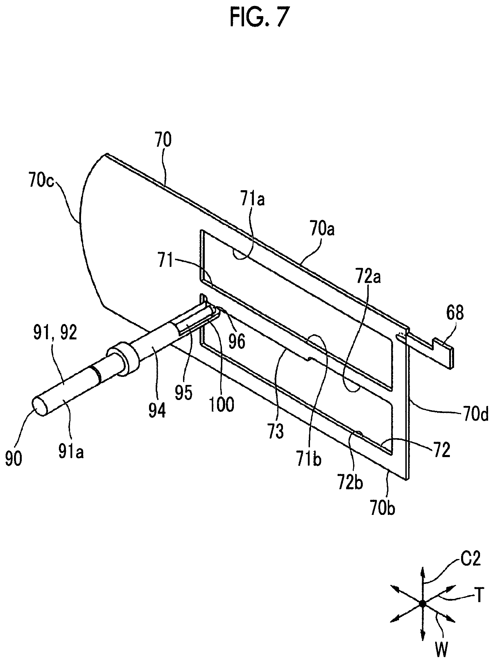

Fig. 7 is a perspective view illustrating a leaf 70 and the drive device 90 that drives the leaf 70.

As illustrated in Figs. 6 and 7, the leaf 70 is formed so that a linear upper edge 70a and a linear lower edge 70b are parallel to each other. As illustrated in Fig. 6, the upper edge 70a is arranged to face the upper surface 61a at a distance therefrom within the leaf housing part 62. The lower edge 70b is arranged to face the lower surface 61b at a distance therefrom within the leaf housing part 62. The leaf 70 is formed so that a front edge 70c that faces the central part of the frame 61 in the width direction W within the leaf housing part 62 swells in a circular-arc shape. Additionally, in the leaf 70, a rear edge 70d that faces the outside of the frame 61 in the width direction W within the leaf housing part 62 is formed in the shape of a straight line orthogonal to the upper edge 70a and the lower edge 70b.

A pair of two leaf groups 70G and 70G arranged to face each other with the central part in the width direction W interposed therebetween within the leaf housing part 62 are arranged so that the front edge 70c of each leaf faces an area between the opening 63 of the upper surface 61a and the opening 63 of the lower surface 61b in the frame 61.

Slits 71 and 72 are formed in each leaf 70 so as to pass therethrough in the thickness direction T. The slits 71 and 72 are respectively formed continuously in a direction in which the front edge 70c and the rear edge 70d of the leaf 70 are connected together. The slits 71 and 72 are formed side by side at an interval in the direction in which the upper edge 70a and the lower edge 70b of the leaf 70 are connected together. The slits 71 and 72 are formed at positions shifted further toward the rear edge 70d side than the front edge 70c so as not to be irradiated with the radiation S2 that enters the leaf housing part 62 of the frame 61 from the opening 63 of the upper surface 61a of the frame 61.

In each leaf 70, a rack gear 73 that is continuous along the width direction W is formed in at least one of the upper side 71a or 72a and the lower side 71b or 72b of the slit 71 or 72. Here, in the leaf group 70G, the rack gears 73 of the leaves 70 and 70 adjacent to each other in a direction in which the plurality of leaves 70 are placed side by side are formed at mutually different sides among the upper sides 71a and 72a and the lower sides 71b and 72b of the slits 71 and 72. By adopting such a configuration, pinion gears 96 that mesh with the rack gear 73 can be prevented from interfering with each other between the leaves 70 and 70 adjacent to each other.

Each of the plurality of leaves 70 that constitute each leaf group 70G are supported by the frame 61 so as to be advanceable and retractable in a direction orthogonal to the thickness direction T and a direction in which the front edge 70c and the rear edge 70d are connected, that is, along the width direction W. For this reason, a plurality of sliding supporting members 66 are provided in the width direction W at intervals at an upper part and a lower part of each leaf group 70G in the frame 61. In this embodiment, two sliding supporting members on a central part side and on an outer peripheral side, respectively, of the frame 61 in the width direction W, that is, a total of four sliding supporting members 66 including are provided in each of the upper part and the lower part of each leaf group 70G.

As illustrated in Figs. 5 and 6, in each of the sliding supporting members 66, a plurality of supporting rollers 66b are rotatably mounted on a shaft 66a fixed to the frame 61. The plurality of supporting rollers 66b are provided at positions corresponding to the respective leaves 70 that constitute the leaf group 70G.

As illustrated in Fig. 6, the supporting rollers 66b of at least two sliding supporting members 66 abut against the upper edge 70a and the lower edge 70b of each leaf 70. Accordingly, each leaf 70 is individually supported by the frame 61 so as to be advanceable and retractable along the width direction W.

Here, in the leaf group 70G, the supporting rollers 66b of mutually different sliding supporting members 66 among the plurality of sliding supporting members 66 may abut against each other in the leaves 70 and 70 adjacent to each other in the thickness direction T in which the plurality of leaves 70 are placed side by side. By adopting such a configuration, the supporting rollers 66b can be prevented from interfering with each other between the leaves 70 and 70 adjacent to each other.

The frame 61 includes a stopper 68 in order to restrict the amount of movement of each leaf 70 to the rear edge 70d side in the width direction W.

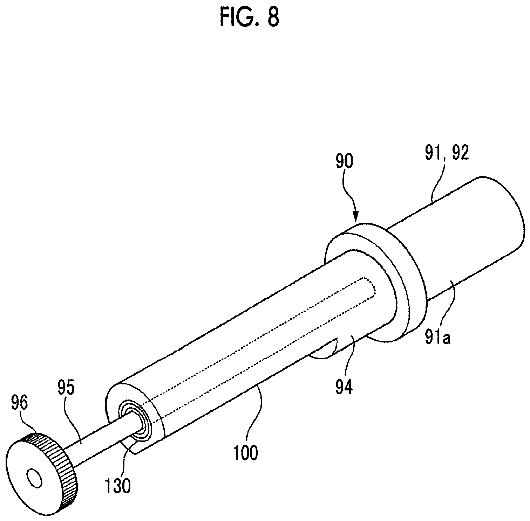

Fig. 8 is a perspective view illustrating the configuration of the drive device 90.

As illustrated in Fig. 7, the drive device 90 is provided to correspond to each of the plurality of leaves 70. As illustrated in Figs. 7 and 8, the drive device 90 includes a motor (driving unit) 91, a shaft 95, and a pinion gear (advance and retraction driving section) 96.

The motor 91 is connected to a base end that is a first end of the shaft 95 in an axis direction. The motor 91 rotates the shaft 95 around its axis. As illustrated in Fig. 5, the motor 91 is supported by a base plate 65 serving as an attachment surface provided along the side surface 61c or 61d of the frame 61.

The motor 91 of the drive device 90 that drives half of the leaves 70 on a side near the side surface 61c among the plurality of leaves 70 that constitute the leaf group 70G is supported by the base plate 65 provided on one side surface 61c of the frame 61. The motor 91 of the drive device 90 that drives half of the leaves 70 on a side near the side surface 61d among the plurality of leaves 70 that constitute the leaf group 70G is supported by the base plate 65 provided on the other side surface 61d of the frame 61.

As illustrated in Fig. 7, the shaft 95 is provided so as to extend in the thickness direction T of the leaves 70. As illustrated in Figs. 6 and 7, the shaft 95 is inserted into the slits 71 or 72 of the plurality of leaves 70 of the leaf group 70G. As illustrated in Figs. 7 and 8, the pinion gear 96 is provided at a tip that is a second end of the shaft 95 in the axis direction. The pinion gear 96 meshes with the rack gear 73 that is formed in any one of the upper sides 71a and 72a and the lower sides 71b and 72b of the slits 71 and 72, which are portions of the leaf 70.

The drive device 90 is further provided with a rotary encoder 92 and a cover 94.

The rotary encoder 92 measures the rotational amount of the shaft 95, and outputs the measurement result to the control apparatus 12.

The cover 94 is formed in a hollow tubular shape. The cover 94 is provided integrally with a housing 91a of the motor 91. The end of the cover 94 opposite to the motor 91 is provided with a bearing 130. The cover 94 has the shaft 95 inserted therethrough. The shaft 95 is rotatably supported by the bearing 130. The cover 94 supports the shaft 95 with the bearing 130 at a position apart from the motor 91. Accordingly, the shaft 95 is prevented from being deformed due to its own weight or the like, and even when the motor 91 and the leaf 70 are separated from each other, the pinion gear 96 reliably meshes with the rack gear 73.

A cutout 100 is formed in a portion of the cover 94 in the circumferential direction. The cutout 100 prevents all interference with other leaves 70 arranged closer to the motor 91 side than a leaf 70 having the rack gear 73 with which the pinion gear 96 of the shaft 95 inserted through this cover 94 meshes.

In such a drive device 90, the motor 91 is driven by the control of the control apparatus 12, and rotates the shaft 95. If the shaft 95 rotates, the pinion gear 96 rotates with the shaft 95, and the rotary power thereof is transmitted to the rack gear 73. Then, the leaf 70 provided with the rack gear 73 advances and retracts along the width direction W.

In this way, the respective leaves 70 that constitute the leaf group 70G are advanced and retracted along the width direction W in each of the pair of two leaf groups 70G. A portion of the radiation S2 that has been incident from the opening 63 of the upper surface 61a of the frame 61 is shielded by the leaves 70 of the leaf groups 70G on both sides. Accordingly, the therapeutic radiation Sr having the shape of a predetermined irradiation field is produced by the multileaf collimator 60A.

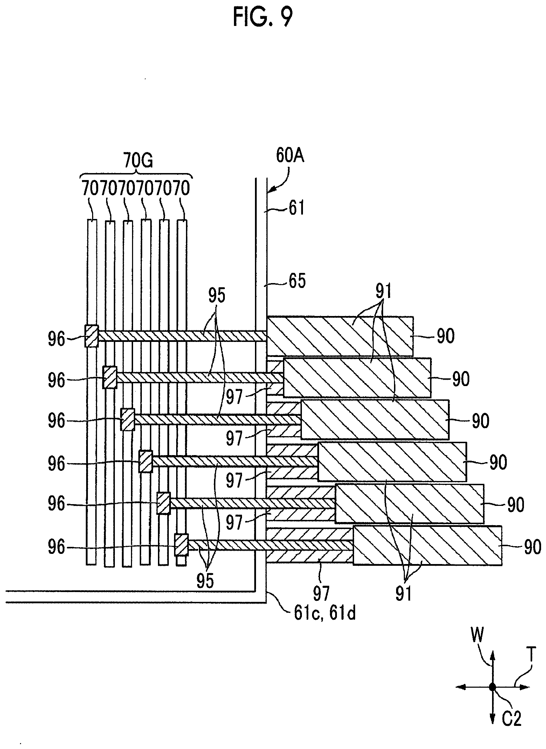

Fig. 9 is a sectional view illustrating the arrangement structure of the drive devices 90 with respect to the leaves 70.

As illustrated in Fig. 9, the plurality of drive devices 90 are respectively provided to correspond to the plurality of leaves 70 that constitute the leaf group 70G. Therefore, the positions of the pinion gears 96 provided at the tips of the shafts 95 are different from each other in the thickness direction T of the leaves 70, in the respective drive devices 90 that drive the plurality of leaves 70. The lengths of the plurality of shafts 95 are made the same as each other in the respective drive devices 90 that drive the plurality of leaves 70. For this reason, the plurality of motors 91 are arranged so that the positions thereof in the thickness direction T are shifted from each other according to the respective tips of the plurality of shafts 95, that is, the positions of the pinion gears 96.

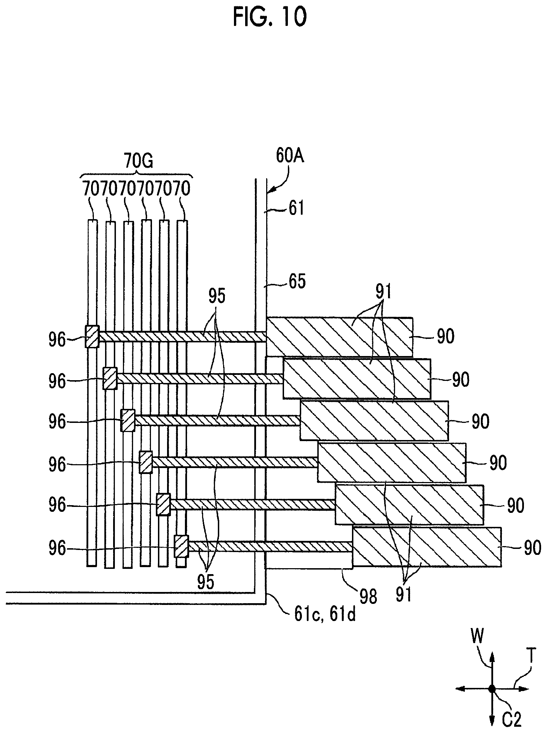

In this embodiment, the motor 91 of each drive device 90 is provided in a base plate 65 of the side surface 61c or 61d of the frame 61 via a spacer 97. The spacer 97 is tubular, and has the shaft 95 and the cover 94 inserted therethrough. In addition, in Fig. 9, illustration of the cover 94 is omitted for the sake of convenience of illustration (in the following, the same also applies to Figs. 10 to 12).

The thicknesses of the spacers 97 in the thickness direction T are set in a plurality of steps. Accordingly, the frame 61 is made to support the drive devices 90 while the positions of the plurality of motors 91 in the thickness direction T are shifted from each other, using the shafts 95 of the same length.

In the radiation therapy system 10 as described above, therapy is performed as follows.

First, a user fixes the patient 200 to the table 28 of the radiation therapy apparatus 20 in a posture indicated by therapeutic planning input to the therapy planning apparatus 11.

The control apparatus 12 actuates the swiveling driving mechanism (not illustrated) and a gantry drive device (not illustrated), and swivels the ring frame 21 and the traveling gantry 22 around the central axes C1 and C2. The radiation irradiation device 24 is moved so that the therapeutic radiation Sr is radiated the position of an affected part of the patient 200 at an irradiation angle indicated by the therapeutic planning. Additionally, the control apparatus 12 makes each leaf 70 advanced and retracted by the drive device 90 so that the shape of the irradiation field of the therapeutic radiation Sr is changed to a shape indicated by the therapeutic planning input to the therapy planning apparatus 11 in the multileaf collimator 60A.

Thereafter, the therapeutic radiation Sr of a dose indicated by the therapeutic planning input to the therapy planning apparatus 11 is radiated to the affected part of the patient 200, using the radiation irradiation device 24.

According to the multileaf collimator 60A of the above-described embodiment, the lengths of the plurality of shafts 95 are made the same as each other between the drive devices 90 that respectively drive the plurality of leaves 70 constituting the leaf group 70G. Moreover, the plurality of motors 91 are arranged so that the positions thereof in the thickness direction T are shifted from each other according to the positions of the pinion gears 96 of the respective tips of the plurality of shafts 95.

By adopting such a configuration, the lengths of the shafts 95 corresponding to the plurality of leaves 70, respectively, can be unified. As a result, the type of parts that constitute the multileaf collimator 60A can be reduced. Additionally, since it is not necessary to prepare the shafts 95 having lengths according to the positions of the respective leafs 70 at the time of repair, the number of parts to be stocked can be reduced.

Additionally, according to the multileaf collimator 60A of the above-described embodiment, the motors 91 are provided on the side surface 61c or 61d that is an outer peripheral surface of the frame 61 via the spacers 97, and the thicknesses of the spacers 97 in the thickness direction T are set in a plurality of steps.

By adopting such a configuration, the plurality of motors 91 are arranged so that the positions thereof in the thickness direction T are shifted from each other according to the positions of the respective tips of the plurality of shafts 95 of which the lengths are united.

As a result, the number of types of parts of the multileaf collimator 60A can be reduced, and it is possible to suppress manufacture costs and maintenance costs.

Moreover, the manufacture costs and maintenance costs of the entire radiation therapy apparatus 20 and the entire radiation therapy system 10 can be suppressed by adopting the multileaf collimator 60A to form the radiation therapy apparatus 20 and the radiation therapy system 10.

### (Modification Example of First Embodiment)

In the first embodiment, the motors 91 are provided on the side surface 61c or 61d of the frame 61 via the spacers 97. However, the invention is not limited to this. If the plurality of motors 91 are arranged so that the positions thereof in the thickness direction T are shifted from each other according to the positions of the respective tips of the plurality of shafts 95 of which the lengths are united, it is possible to adopt, for example, another configuration as illustrated below.

Fig. 10 is a sectional view illustrating a modification example of the arrangement structure of the drive devices 90 with respect to the leaves 70.

As illustrated in this Fig. 10, pedestal parts 98 to which the motors 91 are fixed are formed integrally with the base plate 65 provided on the side surface 61c and 61d that is the outer peripheral surfaces of the frame 61. The pedestal parts 98 are each formed in the shape of a step, and the heights thereof in the thickness direction T may be set in a plurality of steps corresponding to the positions of the respective motors 91 in the thickness direction T.

By adopting such a configuration, the plurality of motors 91 are arranged so that the positions thereof in the thickness direction T are shifted from each other according to the positions of the pinion gears 96 of the respective tips of the plurality of shafts 95 of which the lengths are united. Accordingly, similar to a case where the spacers 97 are used, the number of types of parts of the multileaf collimator 60A can be reduced, and it is possible to suppress manufacture costs and maintenance costs. In this case, since it is not necessary to use the spacers 97, the number of parts that constitute the multileaf collimator 60A can be reduced.

### (Second Embodiment)

Next, a second embodiment of the multileaf collimator related to the invention will be described. A multileaf collimator 60B illustrated in the second embodiment is different from the multileaf collimator 60A of the first embodiment in terms of the setting of the lengths of the shafts 95. Therefore, in the description of the second embodiment, description will be made with the same reference numerals given to the same parts as those of the first embodiment, and duplication description will be omitted. That is, the description about the overall configuration of the multileaf collimator 60B and the configurations of the radiation therapy apparatus 20 and the radiation therapy system 10 that are the same as the configuration described in the first embodiment will be omitted.

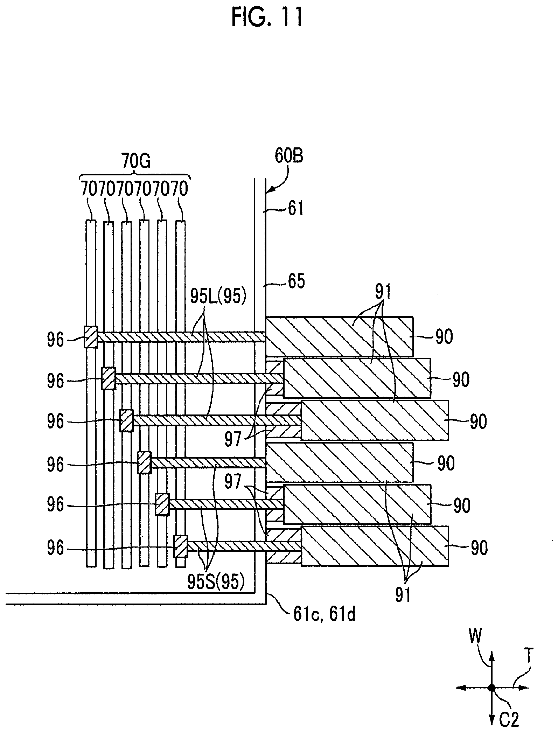

Fig. 11 is a sectional view illustrating the arrangement structure of the drive devices with respect to the leaves of the multileaf collimator 60B in the second embodiment.

As illustrated in Fig. 11, in the multileaf collimator 60B, the drive devices 90 are respectively provided to correspond to the plurality of leaves 70 that constitute the leaf group 70G.

The lengths of the plurality of shafts 95 are made into two long and short types between the respective drive devices 90 that drive the plurality of leaves 70.

The leaves 70 near the center in the direction (thickness direction T) in which the leaves 70 are placed side by side among the plurality of leaves 70 that constitute the leaf group 70G are driven by the drive devices 90 using shafts 95L with a longer length. The leaves 70 near the outer periphery in the direction (thickness direction T) in which the leaves 70 are placed side by side among the plurality of leaves 70 that constitute the leaf group 70G are driven by the drive devices 90 using shafts 95S with a shorter length than the shafts 95L.

In this case, although the plurality of motors 91 are arranged so that the positions thereof in the thickness direction T are shifted from each other, the spacers 97 may be used as illustrated in Fig. 11, or the pedestal parts 98 may be used as illustrated in Fig. 10.

In the multileaf collimator 60B of this embodiment, the shafts 95 of the plurality of drive devices 90 that drive the plurality of leaves 70, respectively, are set to two types of shafts including the shafts 95L with a longer length and the shafts 95S with a shorter length. Therefore, the lengths of the shafts 95 can be made the same as each other in the plurality of drive devices 90 using the longer shafts 95L and the plurality of drive devices 90 using the shorter shafts 95S, respectively. Accordingly, the number of the types of parts that constitute the multileaf collimator 60B can be reduced. Additionally, by setting the shafts 95 to the two types, the types of the spacers 97 or the pedestal parts 98 for shifting the plurality of motors 91 so that the positions thereof in the thickness direction T from each other can also be reduced.

Moreover, since the shorter shafts 95S are used for the leaves 70 near the outer periphery in the direction (thickness direction T) in which the leaves 70 are placed side by side among the plurality of leaves 70 that constitute the leaf group 70G, the projection dimensions of the motors 91 from the side surface 61c or 61d of the frame 61 can be suppressed to be small. Accordingly, the size of the multileaf collimator 60B can be reduced. As a result, the degree of freedom of operation of the radiation irradiation device 24 within the ring frame 21 and the traveling gantry 22 can be improved.

In the second embodiment, the shafts 95 of the plurality of drive devices 90 are set to two types of shafts including the shafts 95L with a longer length and the shafts 95S with a shorter length. However, the shafts can be set to three or more types of shafts. In this case, in at least the plurality of shafts 95, the lengths of the shafts 95 are made the same as each other.

### (Third Embodiment)

Next, a third embodiment of the multileaf collimator related to the invention will be described. A multileaf collimator 60C illustrated in the third embodiment is different from the multileaf collimator 60B of the second embodiment in terms of the setting of the lengths of the shafts 95. Therefore, in the description of the third embodiment, the same reference numerals will be given to the same parts, and duplication description will be omitted. That is, differences from the second embodiment will mainly be described, and the description of the overall configuration of the multileaf collimator 60C and the configurations of the radiation therapy apparatus 20 and the radiation therapy system 10 that are the same as the configurations described in the first and second embodiments will be omitted.

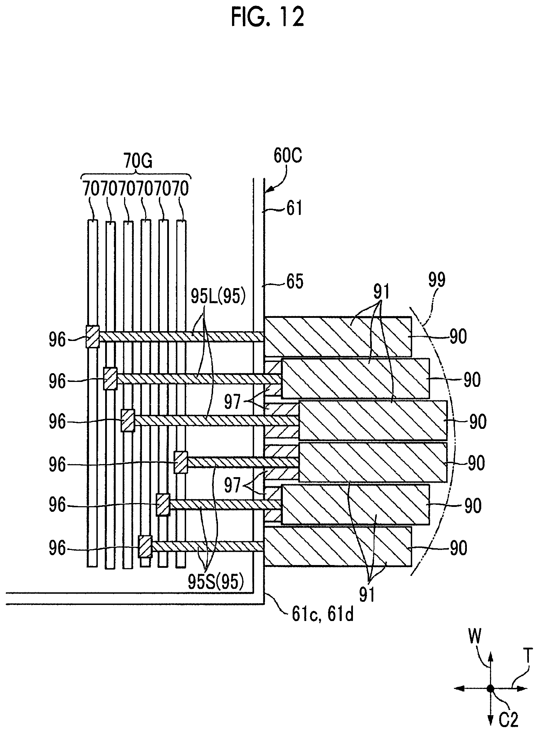

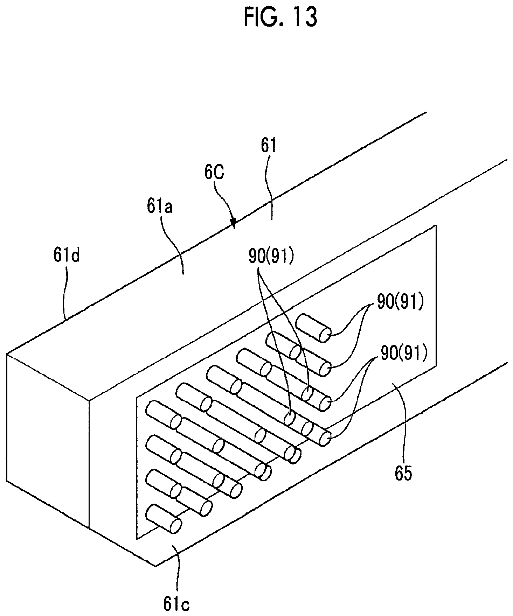

Fig. 12 is a sectional view illustrating the arrangement structure of the drive devices with respect to the leaves of the multileaf collimator in the third embodiment. Fig. 13 is a perspective view of main parts of the multileaf collimator.

As illustrated in Figs. 12 and 13, in the multileaf collimator 60C, the drive devices 90 are respectively provided to correspond to the plurality of leaves 70 that constitute the leaf group 70G.

The plurality of drive devices 90 provided to correspond to the plurality of leaves 70, respectively, are arranged so that the projection dimensions thereof in the thickness direction T from the side surface 61c or 61d become larger gradually toward the central side. More specifically, in the side surface 61c or 61d of the frame 61, the projection dimensions of the motors 91 in the thickness direction T are increased gradually toward an outer side in the direction of the central axis C2 and from an outer side in the width direction W toward central sides in the respective directions. That is, the plurality of motors 91 supported by the base plate 65 are arranged so that the projection dimensions thereof become larger toward the outside of the base plate 65 and the projection dimensions become larger toward the center sides.

Here, the shafts 95 of the respective drive devices 90 may be unified into the same length as in the configuration illustrated in the first embodiment, or may be unified into the plurality of types of lengths as in the configuration illustrated in the second embodiment. In the example of Fig. 12, similar to the configuration illustrated in the second embodiment, the plurality of drive devices 90 may be configured using the shafts 95L and 95S having two types of lengths.

Additionally, in order to arrange the plurality of motors 91 so that the positions thereof in the thickness direction T are shifted from each other, the spacers 97 may be used as illustrated in Fig. 12, or the pedestal parts 98 may be used as illustrated in Fig. 10.

According to the multileaf collimator 60C of this embodiment, in the side surface 61c or 61d of the frame 61, the projection dimensions of the motors 91 become smaller toward the outer peripheral side of the plurality of drive devices 90. Therefore, the size of the multileaf collimator 60B can be reduced. As a result, the degree of freedom of operation of the radiation irradiation device 24 within the ring frame 21 and the traveling gantry 22 can be improved.

Additionally, in the side surface 61c or 61d of the frame 61, the projection dimensions of the motors 91 become gradually larger from the outside of the plurality of drive devices 90 toward the central side thereof. Therefore, for example, in the case of maintenance, the motors 91 on the outside do not easily cause an obstruction, even in the motors 91 on the central side. As a result, workability in which a worker's hand easily reaches the motors 91 can be improved.

Moreover, when the plurality of drive devices 90 are covered with a cover member 99 (refer to Fig. 12) from the outside, a central part of the cover member 99 can be formed in a smooth curved surface so as to protrude gradually. Therefore, degradation of design by the projection of the motors 91 can be prevented.

### (Other Embodiments)

In addition, the invention is not limited to the above-described embodiments, and design changes can be made without departing from the concept of the invention.

For example, the configurations of the respective parts of the multileaf collimators 60A, 60B, and 60C can be appropriately changed to configurations other than those described above. For example, instead of the pinion gears 96, the leaves 70 may be advanced and retracted by pressing rollers against the upper sides 71a and 72a and the lower sides 71b and 72b of the slits 71 and 72 and rotationally driving the rollers with the motors 91.

Moreover, other arbitrary configurations may be adopted regarding the configurations of the respective parts the radiation therapy apparatus 20 and the radiation therapy system 10 other than the multileaf collimators 60A and 60B and 60C.

### Industrial Applicability

By making the lengths of the shafts the same as each other in at least some of the plurality of shafts, the number of types of parts of the multileaf collimator can be reduced, and it is possible to suppress manufacture costs and maintenance costs.

### Reference Signs List

10: RADIATION THERAPY SYSTEM
11: THERAPY PLANNING APPARATUS
12: CONTROL APPARATUS
20: RADIATION THERAPY APPARATUS
21: RING FRAME
21a: LOWER END
22: TRAVELING GANTRY
22a: INNER PERIPHERAL SURFACE
24: RADIATION IRRADIATION DEVICE
25: ROTATING SHAFT
26A: RADIATION SOURCE
27A, 27B: SENSOR ARRAY
28: TABLE
28a: UPPER SURFACE
29: TABLE DRIVE DEVICE
51: ELECTRON BEAM ACCELERATOR
52: X-RAY TARGET
53: PRIMARY COLLIMATOR
53h: THROUGH-HOLE
54: FIELD FLATTENING FILTER
54a: PROJECTION
55: SECONDARY COLLIMATOR
55h: THROUGH-HOLE
60A, 60B, 60C: MULTILEAF COLLIMATOR
61: FRAME
61a: UPPER SURFACE
61b: LOWER SURFACE
61c, 61d: SIDE SURFACE
62: LEAF HOUSING PART
63: OPENING
65: BASE PLATE
66: SLIDING SUPPORTING MEMBER
66a: SHAFT
66b: SUPPORTING ROLLER
68: STOPPER
70: LEAF
70G: LEAF GROUP
70a: UPPER EDGE
70b: LOWER EDGE
70c: FRONT EDGE
70d: REAR EDGE
71, 72: SLIT
71a, 72a: UPPER SIDE
71b, 72b: LOWER SIDE
73: RACK GEAR
90: DRIVE DEVICE
91: MOTOR (DRIVING UNIT)
91a: HOUSING
92: ROTARY ENCODER
94: COVER
95, 95L, 95S: SHAFT
96: PINION GEAR (ADVANCE AND RETRACTION DRIVING SECTION)
97: SPACER
98: PEDESTAL PART
99: COVER MEMBER
101: DIAGONIZING X-RAY
130: BEARING
200: PATIENT
C0: ISOCENTER
C1: CENTRAL AXIS
C2: CENTRAL AXIS
S0: ELECTRON BEAM
S1, S2: RADIATION
Sr: THERAPEUTIC RADIATION

## Claims

1. A multileaf collimator comprising:
two leaf groups (70G) each includinga plurality of leaves (70) that extend in a first direction, each leave including at least one slit (71, 72) extending in the first direction and formed so as to pass therethrough;
a frame (61) that supports the plurality of leaves (70) so that the plurality of leaves (70) are individually advanceable and retractable in a state where the plurality of leaves (70) are placed side by side in a second direction perpendicular to the first direction, said two leaf groups (70G) being arranged within the frame (61) so as to face each other with a central part of the frame in the first direction interposed therebetween;
a plurality of shafts (95) that extend in the second direction passing through said slits (71) of the leaves (70) and are provided to correspond to the plurality of leaves (70), respectively; **characterised by**:
a plurality of driving units (91) that are respectively connected to first ends of the plurality shafts (95) in directions of axial lines and rotate the shafts around the axial lines; and
advance and retraction driving sections (96) that are provided at second ends of the shafts (95) in the directions of the axial lines, contact portions on at least one of the upper side (71a, 72a) and the lower side (71b, 72b) of the slits (71, 72)of the leaves (70), and advance and retract the leaves (70) in the first direction by the rotation of the shafts (95) around the axial lines, wherein at least some of the plurality of shafts (95) corresponding to leaves (70) constituting the same leave group (70G) have the same shaft length as each other, and wherein the plurality of driving units (91) corresponding to leaves (70) constituting the same leave group (70G) are arranged so that the positions thereof in the second direction are shifted from each other according to the positions of the second ends of the plurality of shafts (95) having the same length, wherein at least some of the plurality of driving units (91) are supported via spacers (97) with respect to the frame, and
wherein the thicknesses of the spacers in the second direction are set in a plurality of steps according to the positions of the driving units (91) in the second direction.

2. The multileaf collimator according to Claim 1,
wherein the frame includes pedestal parts (98) that fix the driving units (91), and
wherein the heights of the pedestal parts (98) of the second direction are set in a plurality of steps according to the positions of the driving units (91) in the second direction.

3. The multileaf collimator according to any one of Claims 1 to 2,
wherein the plurality of shafts (95) includes shafts having at least two types of length.

4. The multileaf collimator according to any one of Claims 1 to 3,
wherein the driving units (91) are arranged so that the projection dimensions thereof from the frame in the second direction become gradually larger from the outside of an attachment surface of the frame to which the plurality of driving units (91) are attached toward a central side of the attachment surface.

5. A radiation therapy apparatus comprising:
the multileaf collimator (60A, 60B, 60C) according to any one of Claims 1 to 4; and
a radiation irradiation device (24) that radiates radiation.

6. A radiation therapy system comprising:
the radiation therapy apparatus (20) according to Claim 5; and
a control apparatus (12) that controls the operation of the radiation therapy apparatus (20).

## Patentansprüche

1. Lamellenkollimator, umfassend:
zwei Blattgruppen (70G), die jeweils eine Vielzahl von Blättern (70) umfassen, die sich in einer ersten Richtung erstrecken, wobei jede mindestens einen Schlitz (71, 72) umfasst, der sich in der ersten Richtung erstreckt und als Durchbruch ausgebildet ist;
einen Rahmen (61), der die Mehrzahl von Blättern (70) trägt, so dass die Mehrzahl von Blättern (70) in einem Zustand, in dem die Mehrzahl von Blättern (70) nebeneinander angeordnet sind, in einer zweiten, senkrecht der ersten Richtung verlaufenden Richtung einzeln vorschiebbar und zurückziehbar sind, wobei die zwei Blattgruppen (70G) innerhalb des Rahmens (61) so angeordnet sind, dass sie einander gegenüberliegen, wobei ein mittlerer Teil des Rahmens in der ersten Richtung zwischen ihnen angeordnet ist;
eine Vielzahl von Wellen (95), die sich in der zweiten Richtung erstrecken, die durch die Schlitze (71) der Blätter (70) verlaufen, und die so bereitgestellt sind, dass sie jeweils der Mehrzahl von Blättern (70) entsprechen;
**gekennzeichnet durch:**
eine Mehrzahl von Antriebseinheiten (91), die jeweils mit ersten Enden in Richtungen von ihren Achsen der Vielzahl von Wellen (95) verbunden sind und die Wellen um die Achsen drehen; und Vorschub- und Rückzugantriebsabschnitte (96), die an zweiten Enden in den Richtungen ihrer Achsen der Wellen (95) vorgesehen sind, mit Abschnitten an mindestens einer der oberen Seite (71a, 72a) und der unteren Seite (71b, 72b) der Schlitze (71, 72) der Blätter (70) in Eingriff stehen, und die Blätter (70) in der ersten Richtung durch die Drehung der Wellen (95) um deren Achsen Vorschieben und Zurückziehen,
wobei mindestens einige der Vielzahl von Wellen (95), die Blättern (70) entsprechen, die die gleiche Blattgruppe (70G) bilden, die gleiche Schaftlänge aufweisen und
wobei die Mehrzahl von Antriebseinheiten (91), die Blättern (70) entsprechen, die die gleiche Blattguppe (70G) bilden, so angeordnet sind, dass ihre Positionen in der zweiten Richtung entsprechend den Positionen der zweiten Enden der Mehrzahl von Wellen (95) gleicher Länge gegeneinander verschoben sind,
wobei zumindest einige aus der Mehrzahl von Antriebseinheiten (91) über Abstandshalter (97) relativ zum Rahmen abgestützt sind,
wobei die Dicken der Abstandshalter in der zweiten Richtung in einer Vielzahl von Stufen entsprechend den Positionen der Antriebseinheiten (91) in der zweiten Richtung festgelegt sind.

2. Lamellenkollimator nach Anspruch 1,
wobei der Rahmen Sockelteile (98) umfasst, die die Antriebseinheiten (91) fixieren, und
wobei die Höhen der Sockelteile (98) in der zweiten Richtung in einer Vielzahl von Stufen entsprechend den Positionen der Antriebseinheiten (91) in der zweiten Richtung festgelegt sind.

3. Lamellenkollimator nach einem der Ansprüche 1 bis 2,
wobei die Mehrzahl von Wellen (95) Wellen umfasst, die mindestens zwei Längentypen entsprechen.

4. Lamellenkollimator nach einem der Ansprüche 1 bis 3,
wobei die Antriebseinheiten (91) so angeordnet sind, dass ihre von dem Rahmen in der zweiten Richtung von der Außenseite einer Befestigungsfläche des Rahmens, an dem die Mehrzahl von Antriebseinheiten (91) angebracht sind, vorspringenden Abmessungen zu einer Mitte der Befestigungsfläche hin allmählich größer werden.

5. Strahlentherapiegerät, umfassend:
den Lamellenkollimator (60A, 60B, 60C) nach einem der Ansprüche 1 bis 4; und
eine Strahlungsbestrahlungsvorrichtung (24), die Strahlung ausstrahlt.

6. Ein Strahlentherapiesystem, umfassend:
das Strahlentherapiegerät (20) gemäß Anspruch 5; und
eine Steuervorrichtung (12), die den Betrieb der Strahlentherapievorrichtung (20) steuert.

## Revendications

1. Collimateur à lames multiples comprenant :
deux groupes de lames (70G) incluant chacun une pluralité de lames (70) qui s'étendent dans un premier sens, chaque lame incluant au moins une fente (71, 72) s'étendant dans le premier sens et formée de façon à passer à travers celle-ci ;
un châssis (61) qui supporte la pluralité de lames (70) de telle manière que la pluralité de lames (70) peuvent être individuellement avancées et rétractées dans un état où la pluralité de lames (70) sont placées côte à côte dans un deuxième sens perpendiculaire au premier sens, lesdits deux groupes de lames (70G) étant agencés à l'intérieur du châssis (61) de façon à se faire face l'un l'autre avec une partie centrale du châssis dans le premier sens interposée entre ceux-ci ;
une pluralité d'arbres (95) qui s'étendent dans le deuxième sens en passant à travers lesdites fentes (71) des lames (70) et sont prévus de façon à correspondre à la pluralité de lames (70), respectivement ;
**caractérisé par** :
une pluralité d'unités d'entraînement (91) qui sont respectivement connectées à des premières extrémités de la pluralité d'arbres (95) dans des sens de lignes axiales et font tourner les arbres autour des lignes axiales ; et
des sections d'entraînement en avancement et retrait (96) qui sont prévues à des deuxièmes extrémités des arbres (95) dans les sens des lignes axiales, contactent des parties sur au moins un du côté supérieur (71a, 72a) et du côté inférieur (71b, 72b) des fentes (71, 72) des lames (70), et font avancer et se rétracter les lames (70) dans le premier sens par la rotation des arbres (95) autour des lignes axiales,
dans lequel au moins certains de la pluralité d'arbres (95) correspondant à des lames (70) constituant le même groupe de lames (70G) ont la même longueur d'arbre les uns que les autres, et dans lequel la pluralité d'unités d'entraînement (91) correspondant à des lames (70) constituant le même groupe de lames (70G) sont agencées de telle manière que les positions de celles-ci dans le deuxième sens sont décalées les unes par rapport aux autres en fonction des positions des deuxièmes extrémités de la pluralité d'arbres (95) ayant la même longueur, dans lequel au moins certaines de la pluralité d'unités d'entraînement (91) sont supportées par l'intermédiaire d'espaceurs (97) par rapport au châssis, et dans lequel les épaisseurs des espaceurs dans le deuxième sens sont fixées en une pluralité de marches en fonction des positions des unités d'entraînement (91) dans le deuxième sens.

2. Collimateur à lames multiples selon la revendication 1,
dans lequel le châssis inclut des parties de socle (98) qui fixent les unités d'entraînement (91), et
dans lequel les hauteurs des parties de socle (98) du deuxième sens sont fixées en une pluralité de marches en fonction des positions des unités d'entraînement (91) dans le deuxième sens.

3. Collimateur à lames multiples selon l'une quelconque des revendications 1 à 2, dans lequel la pluralité d'arbres (95) inclut des arbres ayant au moins deux types de longueur.

4. Collimateur à lames multiples selon l'une quelconque des revendications 1 à 3, dans lequel les unités d'entraînement (91) sont agencées de telle façon que les dimensions de projections de celles-ci depuis le châssis dans le deuxième sens deviennent graduellement plus grandes de l'extérieur d'une surface de fixation du châssis à laquelle la pluralité d'unités d'entraînement (91) sont fixées vers un côté central de la surface de fixation.

5. Appareil de radiothérapie comprenant :
le collimateur à lames multiples (60A, 60B, 60C) selon l'une quelconque des revendications 1 à 4 ; et
un dispositif d'irradiation de rayonnement (24) qui irradie un rayonnement.

6. Système de radiothérapie comprenant :
l'appareil de radiothérapie (20) selon la revendication 5 ; et
un appareil de commande (12) qui commande le fonctionnement de l'appareil de radiothérapie (20).
